# EUROPEAN PATENT APPLICATION

(11) **EP 3 608 422 A1**
(43) Date of publication of application: **12.02.2020**
(21) Application number: 18188099.8
(22) Date of filing: 08.08.2018
(51) Int. Cl.: C12Q 1/6869

(54) **ELECTROCHEMICAL DETECTION OF POLYMERASE REACTIONS BY SPECIFIC METAL-PHOSPHATE COMPLEX FORMATION**

(71) Applicant: Amaryllis Innovation GmbH, 14195 Berlin (DE)
(72) Inventor: Glökler, Jörn, 10783 Berlin (DE)
(74) Representative: CH Kilger Anwaltspartnerschaft mbB

(57) **Abstract**

The present invention relates to determining pyrophosphate by electrochemical means as a result of its binding to and/or precipitation with metal oxides on an electrode. This principle is of particular interest for polymerase catalysed reactions such as nucleic acid sequencing.

## Description

### FIELD OF THE INVENTION

The present invention is in the field of chemistry and biology. Specifically, the invention relates to the electrochemical detection of pyrophosphate and to electrochemical nucleic acid sequencing.

### INTRODUCTION

Nucleic acid polymerase reactions are central to a multitude of sensitive detection methods. Especially many different solutions have been developed for PCR product formation. More specifically, a certain reaction used to identify a DNA sequence termed pyrosequencing (Ahmadian et al. Clin. Chim. Acta. 2006. 363(1-2):83-94) is of great interest. In pyrosequencing the four different nucleotides are added to a polymerase reaction in alternating cycles and the release of pyrophosphate is monitored indirectly.

Currently, two main approaches for the detection of product formation from a polymerase reaction such as a nucleic acid sequencing method exist:
Pyrophosphate is used to generate ATP via sulfurylase and ADPS, which leads to light emission by luciferase (Ahmadian et al. Clin. Chim. Acta. 2006. 363(1-2):83-94). The light is detected and corresponds to a given reaction by polymerase. As a disadvantage, this method utilizes expensive and unstable agents, such as enzymes and ATP-derivates.

Another method is employed by Ion Torrent that measures the polymerase reaction by a change in pH (Rothberg et al. Nature. 2011 Jul 21;475(7356):348-52). The reaction liberates one extra H⁺-ion which is detected by a chemFET or ISFET (see WO2010047804). The advantage of this method is a simple adaptation for integrated circuits which allows considerable miniaturization and parallelization. No extra enzymes other than the polymerase are necessary. However, the reaction needs to occur largely unbuffered and suffers from a low sensitivity.

In other methods pyrophosphate is detected by complexation with certain metal ions. Calcein/Mn²⁺ is a colourless complex, which becomes fluorescent when the manganese is depleted and occupied with magnesium (Tomita et al. Nat Protoc. 2008;3(5):877-82.). Alternatively, pyrophosphate has been used to measure manganese ions in solution by optical means (Takashima et al. J. American Chem. Soc. [Internet]. 2011 Dec 28).

Aluminium ions also form a tight specific complex with pyrophosphate. A fluorescent rhodamine/Al³⁺ complex is depleted of aluminium by the synthesis of pyrophosphate and thus becomes colorless for detection (Lohani et al. Analyst. 2010. 135(8):2079-84). Free, uncomplexed Al(III) can be detected with ISFET (Abbaspour et al. Anal. Chim.Acta. 2010 Mar 3;662(1):76-81.). A chelator-modified silicon-on-insulator field-effect transistor (SOI-FET) with bound Zn²⁺ ions has been shown to detect pyrophosphate liberated by a polymerase reaction directly by the immobilized complex (Credo et al. The Analyst [Internet]. 2012 Jan 19).

Several other metal ions are known to be specifically displaced from complexes (e.g. transferrin) by pyrophosphate, e.g. Fe³⁺, Ga³⁺, In³⁺ (Harris et al. Coordination Chemistry Reviews. 2002. 228(2):237-62), Sn^{2+,} and Zn²⁺ (Cigala et al. Journal of Molecular Liquids. 2012. 165:143-153).

Some lanthanide metal ions such as terbium, europium and ytterbium are known to bind to phosphate species such as triphosphates, DNA, pyrophosphate, and phosphate, albeit with different affinities (Spangler et al. Ann. N. Y. Acad. Sci. 2008. 1130:138-48.). These metal ions can be used either free or in complex with certain ligands that may increase their specificity. So far, these measurements were conducted by using the unique fluorescence properties of lanthanides.

The main approaches suffer from the drawback that they either require expensive and unstable agents, or the reaction is carried out in a largely unbuffered system and has a low sensitivity. Methods based on the formation of metal ion/pyrophosphate complexes require extra hardware such as optical devices or special surface receptors (for the method of Credo *et al.*) which is expensive and hard to implement into existing sequencing units.

Recently, synthetic nucleotide analogues lacking terminal pyrophosphate moieties were successfully tested in polymerase reactions (Herdewijn and Marlière. FEBS Lett. 2012. Jul 16;586(15):2049-56.). The pyrophosphate leaving group was replaced by synthetic compounds that were equally liberated upon base incorporation by various polymerases. Since many of the effective leaving groups are metal ion chelating groups such as aspartic acid, iminodipropionic acid, or iminodiacetic acid, it is conceivable that the detection principle of polymerase reactions by metal ion binding leaving groups using such nucleotide analogues remains the same.

The problem to be solved by the present invention may be formulated as the provision of an inexpensive and simple method for a fast and sensitive detection of nucleic acid polymerase activity with a potential for miniaturization and parallelization. Ideally, the method may be integrated into current systems such as sequencing devices without or with few modifications.

### DEFINITIONS

### Sample

A sample refers to any kind of chemical or biological substance or substance mixture comprising a nucleic acid, wherein the sequence or part of the sequence of the nucleic acid is of interest. A sample may stem from or comprise a prokaryote or an eukaryote, such as an archaeon, a bacterium, a protist, a fungus, a virus, a viroid, a plant, an animal or a synthetic oligonucleotide.

### Nucleic acid

The term (template) nucleic acid is here used in its broadest sense and comprises ribonucleic acids (RNA) and deoxyribonucleic acids (DNA) from all possible sources, in all lengths and configurations, such as double-stranded, single-stranded, circular, linear or branched. All sub-units and sub-types are also comprised, such as monomeric nucleotides, oligomers, plasmids, viral and bacterial nucleic acids, as well as genomic and non-genomic DNA and RNA from animal and plant cells or other eukaryotes or prokaryotes, mRNA (messenger RNA) in processed and unprocessed form, tRNA(transfer RNA), hn-RNA (heterogeneous nuclear RNA), rRNA (ribosomal RNA),LNA (locked nucleic acid), mtRNA (mitochondrial), nRNA (nuclear RNA), siRNA (short interfering RNA), snRNA (small nuclear RNA), snoRNA (small nucleolar RNA), scaRNA (Small Cajal Body specific RNA), microRNA, dsRNA (double-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or as well as all other conceivable nucleic acids.

Sometimes the concentration of the nucleic acid to be sequenced (template nucleic acid) may be too low for sequencing. In this case, the sample may be subjected to an amplification method prior to sequencing. Nucleic-acid amplification can be accomplished by any of the various nucleic-acid amplification methods known in the art, including but not limited to the polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification. In a preferred embodiment the amplification method is selected from the group of polymerase chain reaction (PCR), real-time PCR (rtPCR), helicase-dependent amplification (HDA) and recombinase-polymerase amplification (RPA).

### Primer

The sequence of the primer (molecule) may be a random sequence. However, in cases where part of the sequence of the template nucleic acid is already known the primer can be designed to be complementary to such a sequence. In one embodiment, the primer anneals to either the 3' or the 5' end of the template nucleic acid. The primer may be prepared using any suitable method, such as, for example, the phosphotriester and phosphodiester methods or automated embodiments thereof. In one such automated embodiment diethylophosphoramidites are used as starting materials and may be synthesized as described by Beaucage et al. Tetrahedron Letters. 1981. 22:1859-1862. One method for synthesizing oligonucleotides on a modified solid support is described in U.S. Pat. No. 4,458,006, which is hereby incorporated by reference. It is also possible to use a primer which has been isolated from a biological source (such as a restriction endonuclease digest).
Preferred primers have a length of about 15 - 100, more preferably about 20 - 50, most preferably about 20 - 40 bases.

### Nucleotides

As used herein, the term nucleotides refer to deoxyribonucleoside polyphosphates, such as deoxyribonucleoside triphosphates (dNTPs). Non-limiting examples of such dNTPs are dATP, dGTP, dCTP, dTTP, dUTP, which may also be present in the form of labelled derivatives, for instance comprising a fluorescent label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Deoxyribonucleoside polyphosphates, i.e. nucleotides with more than 3 phosphates, are also utilized by polymerases. In this case the polymerase reaction does not generate pyrophosphate. However, the principle of selecting a metal ion for binding to other phosphates (such as triphosphate) and using it for electrochemical detecting said phosphate can be applied correspondingly.

### Elongation

Nucleic acid elongation herein means the stepwise addition of nucleotides to the growing nucleic acid chain. Elongation is catalyzed by a polymerase. Herein, a polymerase include but are not limited to T7 DNA polymerase, DNA Polymerase γ, Escherichia coli DNA pol I, Thermus aquaticus pol I, Bacillus stearothermophilus pol I, Pol II (bacterial), Phi29 DNA polymerase, Pol B (archaebacterial) such as 9°N DNA polymerase, Pol α, δ, ε and ζ, eukaryotic polymerase pol β, pol σ, pol λ, pol µ, terminal deoxynucleotidyltransferase (TdT), Pol X polymerase and Pol IV.

### Sequencing

Nucleic acid sequencing can consist of determining whether a particular nucleic acid differs in sequence from a reference nucleic acid, confirming the presence of a particular nucleic acid sequence in a sample, determining partial sequence information such as the identity of one or more nucleotides within a nucleic acid, determining the identity and order of nucleotides within a nucleic acid, etc.
Most sequencing methods relate to the sequencing of DNA. Current methods, thus, typically require RNA to be converted to complementary DNA (cDNA) via reverse transcription prior to sequencing. Thus, if RNA is to be sequenced, the RNA may be first reverse transcribed into cDNA.

### Annealing

Annealing refers to the pairing of the primer by hydrogen bonds to a complementary sequence on the template nucleic acid, forming a double-stranded polynucleotide. Annealing may be facilitated by decreasing the temperature.

### Electrode measurements

Electrochemical measurements may be made in an electrochemical cell consisting of two or more electrodes and the electronic circuitry for controlling and measuring the current and the potential. The simplest electrochemical cell uses two electrodes. The potential of one electrode is sensitive to the analyte's concentration, and is called the working electrode or the indicator electrode, herein only electrode. The second electrode, which is called reference electrode, completes the electrical circuit and provides a reference potential against which the working electrode's potential is measured. Ideally the reference electrode's potential remains constant so that one can assign to the working electrode any change in the overall cell potential.

Potentiometry is the field of electroanalytical chemistry in which potential is measured under the conditions of no current flow. The measured potential may then be used to determine the analytical quantity of interest, generally the concentration of some component of the analyte solution. The potential that develops in the electrochemical cell is the result of the free energy change that would occur if the chemical phenomena were to proceed until the equilibrium condition has been satisfied.

A reference electrode is an electrode which has a stable and well-known electrode potential. Reference electrodes are well-known to the skilled person.

### DETAILED DESCRIPTION OF THE INVENTION

The inventors have unexpectedly found that biological reactions which release pyrophosphate may be detected by electrochemical methods via the formation of pyrophosphate/metal oxide complexes and/or precipitates.

The invention relates to an electrochemical nucleic acid sequencing method comprising the steps of:
a. providing a sample comprising a template nucleic acid to be sequenced;
b. annealing a primer molecule to said template nucleic acid, or creating a nick in the non-template strand for a site-specific elongation;
c. carrying out nucleic acid elongation steps with a polymerase on said template in the presence of metal oxides on an electrode, wherein each step comprises the addition of a single type of nucleotide, wherein pyrophosphate is released in a stoichiometric ratio to the number of nucleotides incorporated, and wherein the metal oxides on the electrode are capable of binding to the pyrophosphate released from the nucleotide;
d. determining in said sample the potential, current or charge at the electrode at least twice, wherein between the first time and the second time one or more elongation steps take place or, alternatively, determining the potential, current or charge continuously; and
e. correlating the change in potential, current or charge over the number of elongation steps with the type of nucleotides added, wherein the electrode is covered with aluminium oxide, iron oxide, vanadium oxide, indium oxide, manganese oxide, tin oxide and/or zinc oxide or a mixture of these.

The inventors have astonishingly found that the addition of an additional free metal ion or chelate to the the solution while performing the PPi reaction is not necessary for detection. The PPi will form a detectable complex also at the site of the electrode and directly or indirectly bind the metal oxide present on the the electrode, thereby creating a measurable signal which is directly proportional to the PPi released into the solution. The template from which the PPi is released may be in solution or also bound directly to the electrode. In this embodiment the template sits bound to the electrode comprising the metal oxide.

The present invention is based on the principle that pyrophosphate (PPi; inorganic diphosphate) can be bound and/or precipitated directly in a biological reaction (in a reaction mixture). The binding and/or precipitation decreases the concentration of unbound and/or dissolved (free) PPi, subsequently resulting in a change of signal generation in an electrochemical sensor. The present invention uses the principle that the PPi is not required to bind free, dissolved metal ions, but astonishingly the PPi may bind the metal oxide present on the electrode, where it results in a change of signal. The main advantage is that many free metal ions such as manganese either inhibit or alter the specificity of polymerases.

A typical PPi releasing reaction is a reaction that uses the synthetic activity of a polymerase. For example, DNA polymerase catalyses the polymerization of deoxyribonucleotides (dNTPs) into a DNA strand. The addition of one of the four complementary dNTPs onto the template releases PPi stoichiometrically. This principle can be exploited in that the synthetic activity of the polymerase is determined indirectly over the release of PPi. Free metal ions can be detected by electrochemical methods and thus indirectly indicate the amount of pyrophosphate complex formed by the reaction despite the presence of triphosphates and oligonucleotides. In the present invention however the complex comes into existence at the electrode, where the metal ion or oxide thereof sits attached to the electrode. The pyrophosphate concentration bound to the metal oxide of the electrode results in a corresponding signal change at the electrode. This will occur only if a nucleotide matching to the template is offered to the polymerase. Thus, this technique can be applied to the detection of nucleic acid sequencing reactions. In a preferred embodiment the electrode is functionalized with an inorganic coating, the metal oxide.

The invention relates to the use of an electrode coated with metal oxide, for detecting and or sequencing a nucleic acid or detecting the presence of PPi.

### Sequencing

Accordingly, in a first aspect the present invention relates to an electrochemical nucleic acid sequencing method comprising the steps of (a) providing a sample comprising a template nucleic acid to be sequenced; (b) annealing a primer molecule to said template nucleic acid or introducing a nick at a predetermined site into a double stranded template; (c) carrying out nucleic acid elongation steps with a polymerase on said template in the presence of metal oxides on an electrode, wherein each step comprises the addition of a single type of nucleotide, wherein pyrophosphate is released in a stoichiometric ratio to the number of nucleotides incorporated, and wherein the metal oxides are capable of specifically binding to pyrophosphate; (d) determining in said sample the change of potential, current or charge at an electrode and (e) correlating this change in potential, current or charge over the number of elongation steps with the type of nucleotides added.

To correlate the signal observed with the sequence of the template nucleic acid to be sequenced one may proceed as follows:
A change in the signal may be observed, once a nucleotide is successfully incorporated. Since only one type of nucleotide (adenine A, cytosine C, guanine G, or uracil U/thymine T) is added at the same time, one can deduce the type of incorporated (elongated) nucleotide. Repeating the steps of adding types of nucleotides and observing the signal one may further deduce the whole sequence of the template nucleic acid.

Preferably, a calibration curve is generated by measuring the signal at various known concentrations of metal ions used for binding and/or precipitating pyrophosphate in order to ascertain confidence parameters. For example pyrophosphate may be added in various known concentrations to a solution comprising a metal oxide on an electrode in another experiment. The calibration curve is preferably made in a solution (e.g. a buffer) which is comparable to the test solution (e.g. sequencing buffer).

Preferably, the nucleic acid sequencing method is based on pyrosequencing, reversible-terminator-pyrosequencing, or closed complex formation sequencing.

It is preferred that step d. and optionally step e. is done after each elongation step c. Alternatively, step d. and optionally step e. is done after every second, third, fourth, firth or even more elongation steps c.

The sequencing method according to the invention is preferably based on pyrosequencing. Pyrosequencing is based on the detection of the pyrophosphate (PPi) that is released during DNA polymerization (see, e.g., U.S. Pat. Nos. 6,210,891 and 6,258,568). While avoiding the need for electrophoretic separation, pyrosequencing suffers from a large number of drawbacks that have as yet limited its widespread applicability (Franca et al. Quarterly Reviews of Biophysics. 2002. 35(2):169-200).

In an alternative embodiment the step of sequencing involves reversible-terminator-pyrosequencing. The method is extensively explained in Wu et al. PNAS. 2007. 104(42):16462-16467, which is hereby incorporated by reference.

In yet another embodiment sequencing involves closed complex formation sequencing. The method is described in WO 2007/048033, which is hereby incorporated by reference.

### Polymerase reaction

The inventive concept is particularly advantageous in the case of sequencing a nucleic acid. There are, however, other cases where it is desirable to detect and/or quantify the amount (e.g. concentration) or a change in said amount of PPi with a fast and cost-effective method.

Consequently, the invention relates to a method for detecting a nucleic acid polymerase reaction comprising the steps of (a) providing a sample comprising a nucleic acid; (b) carrying out a nucleic acid polymerase reaction step on said sample in the presence of metal oxides on an electrode, wherein pyrophosphate is released or depleted depending on the type of polymerase reaction, and wherein the metal oxides are capable of specifically binding to pyrophosphate; (c) determining the potential, current or charge at an electrode at least twice, wherein between the first time and the second time one or more nucleic acid polymerase reaction steps take place; and (d) correlating the difference in potential, current or charge over the number of polymerase reaction steps and/or time with the progress of the nucleic acid polymerase reaction.

It is preferred that the nucleic acid polymerase reaction is a synthesis reaction, an amplification reaction and/or a transcription reaction.

It is further preferred that the step of determining the potential, current or charge (step c.) and optionally the step of correlating the difference in potential, current or charge step (step d.) is done after each polymerase reaction step (step b.). Alternatively, step c. and optionally step d. is done after every second, third, fourth, fifth or even more elongation polymerase reaction steps.

As described above, the principle can generally be utilized for assessing whether the concentration of pyrophosphate changes in a sample. The method of detecting the formation or depletion of pyrophosphate in a sample comprises the following steps: (a) providing a sample in which pyrophosphate is formed or depleted; (b) adding to said sample metal oxides on an electrode, wherein the metal oxides are capable of preferentially binding pyrophosphate; (c) determining the potential, current or charge in the sample a first and at least a second time; (d) correlating the difference in potential, current or charge over time with the formation or depletion of pyrophosphate in the sample.

Further, the invention can further be used for detecting the presence and/or the quantity, e.g. concentration, of pyrophosphate in a sample. This method comprises the steps of (a) adding to a sample comprising pyrophosphate a metal oxides on an electrode, wherein the metal oxides are capable of specifically binding pyrophosphate; (b) determining in said sample the potential, current or charge at an electrode; and (c) correlating the potential, current or charge with the amount of pyrophosphate present in said sample.

### Metal oxides

There are a number of different metal oxides that can bind to and/or precipitate pyrophosphate and, thus, be used in the present method. Preferred metal oxides are manganese oxides, vanadium oxides, tin oxides, zinc oxides, copper oxides, molybdenum oxides, aluminium oxides, iron oxides, indium oxides, gallium oxides, zirconium oxides, and lanthanide oxides. More preferred metal oxides of the 4^{th} and 5^{th} period that can form more than one oxide with a different oxidation state such as manganese oxides, vanadium oxides, tin oxides and iron oxides. Most preferred metal ions are manganese oxides, of which manganese dioxide is preferred

### Electrochemical Measurements

The present invention is not restricted to a particular electrochemical technique. For example, potentiometry, controlled-current coulometry, amperometry, controlled-potential coulometry, stripping voltammetry, hydrodynamic voltammetry, polarography and stationary electrode voltammetry, pulse polarography and voltammetry and cyclic voltammetry may be used.

Herein, however, potentiometry is preferred, since it is simple, inexpensive and may be further miniaturized. Furthermore, potentiometry is a quantitative technique. The skilled person will readily understand that and how the principle may be transferred to other electrochemical techniques.

The working electrode must be selected such that it responds to pyrophosphate. This means that e.g. for a potentiometric detection the potential of the electrode depends on the concentration of pyrophosphate. In certain cases it may be advantageous that the electrode is highly selective for the pyrophosphate in the presence of other compounds such as nucleotides. But this may not be necessary in cases where the composition of the solution can be controlled such as in the case of nucleic acid elongation. Preferred working electrodes are electrodes based on manganese oxide. They show a signal which correlates to the concentration of the pyrophosphate in solution.

Metal oxides may change their oxidation state during storage or even during the measurements. It is therefore advantageous to reset the oxidation state of the metal oxide on an electrode to the functional metal oxide before the measurement. Preferably, a reduction is performed by adding a reducing agent to the reaction chamber. More preferably, said reducing agent does not inhibit or alter the properties of the enzyme in the reaction. Most preferably, ascorbic acid is used as a reducing agent.

If pyrophosphate forms avid complexes on the metal oxide, the active surface of the electrode must be regenerated for continued measurements. Such regeneration are preferably performed by adding reducing agents or other compounds that can solubilise pyrophosphate without affecting the activity of the enzyme in the reaction.

As reference electrode, which provides a stable potential, for example a silver/silver chloride or a saturated calomel electrode (SCE) may be used in the inventive method. Preferably, the reference electrode is a silver/silver chloride electrode with a salt bridge. But also miniaturized reference electrode constructions are possible.

In a specific embodiment, the electrode is a manganese oxide electrode and the reference electrode is a silver/silver chloride electrode.

The present method has a number of advantages: The method is simple, cost-effective and may be easily implemented into existing devices. Moreover, since buffer conditions and components during pyrosequencing can be highly controlled, even less specifically binding and/or precipitating metal oxides can be used to monitor the polymerase reaction. As the enzyme may not come in direct contact to the metal oxide, no inhibition or otherwise negative interference with an enzymatic reaction can occur.

### Identification procedure for suitable combination of metal oxide/working electrode

In general, for identifying further working electrodes one may first select suitable metal ions and then chose the corresponding working electrode. The type of metal ion should be selected such that they meet one or more of the following criteria. Ideally, the metal oxides selected meet all criteria: (1) The metal oxides bind and/or precipitate PPi, preferably specifically. In some cases, metal oxides binding to and/or precipitating Phosphate (Pi) may be also used. In this case, a pyrophosphatase should be added to the sample. (2) The metal oxides selected do not or not substantially bind to nucleic acids such as RNA, DNA, NTP, dNTP and proteins or are efficiently blocked/displaced by ions present in the buffer such as magnesium. (3) The metal oxides selected preferably do not affect the chemical composition and structure of DNA and/or RNA. (4) The metal oxides selected do not generate free metal ions affect (e.g. inhibit) the polymerase under typical conditions used in the method. (5) The metal oxides are contacted with the electrode in such a way that slight changes in the concentration or complex formation of metal ions can be measured. The metal oxides are preferably on the electrode but may additionally be added to the solution. For miniaturisation and/or parallelisation it is preferred to employ CMOS chips in which the electrodes are functionalised with a thin layer of metal oxides capable of binding specifically to pyrophosphate.

By way of example, DNA is known to be damaged e.g. by platinum. Hence, such metals do not appear to be suitable if they become soluble. In contrast, tin can be present in at least two oxidation states, i.e. Sn(II) and Sn(IV). It binds more specific to PPi (and Pi) than to ATP. It is assumed that tin does not bind strongly to DNA; it might, however, be necessary to additionally add magnesium ions to the sample. Thus, tin oxide may be an alternative to manganese oxide. Further, PPi (and/or Pi) binding metal oxides are molybdenum Mo, aluminium Al, iron Fe, gallium Ga, indium In, zirconium Zr, vanadium V, and chromium Cr, all of which can be present on the electrode or/and added as mixtures additionally to the reaction.

### Electrochemical nucleic acid sequencing apparatus

The invention further relates to an electrochemical nucleic acid sequencing apparatus comprising: (a) means for carrying out the steps of annealing and elongating a primer molecule to a template nucleic acid to be sequenced; and (b) an electrochemical cell comprising metal oxides being capable of specifically binding to pyrophosphate on a working electrode. In a preferred embodiment the metal oxides are manganese oxides and more preferably the electrode is based on manganese dioxide.

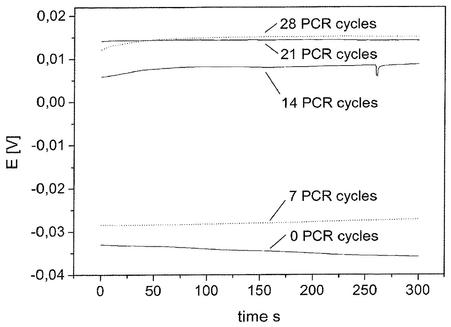

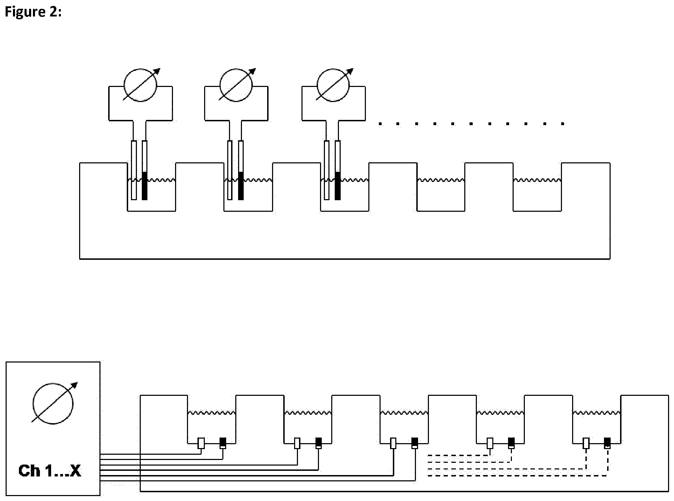

In a preferred arrangement these parts are combined in a small reaction chamber (see Fig. 2).

The means for carrying out the steps of annealing and elongating a primer may comprise a temperature control unit for heating and cooling, such as one or more Peltier elements. It may also comprise means for supplying reagents and buffers to the reaction chamber. Such means can comprise one or more channels (e.g. tubes). Preferably the reagents and buffers can be supplied through different channels, i.e. one channel for each nucleotide or alternatively for all nucleotides, one channel for the sample, one channel for the polymerase, etc. The channels are preferably controllable, e.g. by valves.

## Claims

1. An electrochemical nucleic acid sequencing method comprising the steps of:
providing a sample comprising a template nucleic acid to be sequenced;
a. annealing a primer molecule to said template nucleic acid, or creating a nick in the non-template strand for a site-specific elongation;
b. carrying out nucleic acid elongation steps with a polymerase on said template in the presence of metal oxides on an electrode, wherein each step comprises the addition of a single type of nucleotide, wherein pyrophosphate is released in a stoichiometric ratio to the number of nucleotides incorporated, and wherein the metal oxides on the electrode are capable of preferentially binding to the pyrophosphate released from the nucleotide;
c. determining in said sample the potential, current or charge at an electrode at least twice, wherein between the first time and the second time one or more elongation steps take place or, alternatively, determining the potential, current or charge continuously; and
d. correlating the change in potential, current or charge over the number of elongation steps with the type of nucleotides added, wherein the electrode is covered with aluminium oxide, iron oxide, vanadium oxide, indium oxide, manganese oxide, tin oxide and/or zinc oxide or a mixture of these.

2. The method according to claim 1, wherein the
i) aluminium oxide is aluminium (I) oxide, aluminium (II) oxide or aluminium (III) oxide
ii) the iron oxide is, iron (II) oxide, or iron (III) oxide,
iii) the manganese oxide is manganese (II) oxide, manganese (III) oxide or manganese (IV) oxide
iv) the indium oxide, indium (III) oxide
v) the tin oxide is tin (IV) oxide.
vi) the vanadium oxide is vanadium (V) oxide

3. The method according to claim 1 or 2, wherein the nucleic acid sequencing method is based on pyrosequencing, reversible-terminator-pyrosequencing, or closed complex formation sequencing.

4. The method according to claims 1 or 2, wherein step d. and optionally step e. is done after each elongation step c.

5. A method for detecting a nucleic acid polymerase reaction comprising the steps of:
a. providing a sample comprising a nucleic acid;
b. carrying out a nucleic acid polymerase reaction step on said sample in the presence of metal oxides which are present on an electrode, wherein pyrophosphate is released or depleted depending on the type of polymerase reaction, and wherein the metal oxides on the electrode are capable of specifically binding the pyrophosphate released in the solution;
c. determining in said sample the potential, current or charge at said electrode at least twice, wherein between the first time and the second time one or more nucleic acid polymerase reaction steps take place or, alternatively, determining the potential, current or charge continuously; and
d. correlating the difference in potential, current or charge over the number of polymerase reaction steps and/or time with the progress of the nucleic acid polymerase reaction.

6. The method according to claim 1, wherein the
i) aluminium oxide is aluminium (I) oxide, aluminium (II) oxide or aluminium (III) oxide
ii) the iron oxide is, iron (II) oxide, or iron (III) oxide,
iii) the manganese oxide is manganese (II) oxide, manganese (III) oxide or manganese (IV) oxide
iv) the indium oxide, indium (III) oxide
v) the tin oxide is tin (IV) oxide
vi) the vanadium oxide is vanadium (V) oxide.

7. The method according to claim 4 or 5, wherein the nucleic acid polymerase reaction is a synthesis reaction, an amplification reaction and/or a transcription reaction.

8. The method according to claim 4 to 6, wherein step c. and optionally step d. is done after each polymerase reaction step b.

9. A method of detecting the formation or depletion of pyrophosphate in a sample comprising the steps of:
a. providing a sample in which pyrophosphate is formed or depleted;
b. providing an electrode comprising metal oxides, wherein the metal oxides are capable of specifically binding the pyrophosphate released into the solution;
c. determining in said sample the potential, current or charge at said electrode a first and at least a second time or continuously;
d. correlating the difference in potential, current or charge over time with the formation or depletion of pyrophosphate in the sample.

10. The method according to claim 8, wherein the
i) aluminium oxide is aluminium (I) oxide, aluminium (II) oxide or aluminium (III) oxide
ii) the iron oxide is, iron (II) oxide, or iron (III) oxide,
iii) the manganese oxide is manganese (II) oxide, manganese (III) oxide or manganese (IV) oxide
iv) the indium oxide, indium (III) oxide
v) the tin oxide is tin (IV) oxide
vi) the vanadium oxide is vanadium (V) oxide.

11. A method of electrochemically quantifying pyrophosphate in a sample comprising the steps of:
a. adding to a sample comprising pyrophosphate and an electrode comprising metal oxides, wherein the metal oxides on the electrode are capable of specifically binding pyrophosphate in solution;
b. determining in said sample the potential, current or charge at an electrode; and
c. correlating the potential, current or charge with the amount of pyrophosphate present in said sample.

12. The method according to claim 10, wherein the
i) aluminium oxide is aluminium (I) oxide, aluminium (II) oxide or aluminium (III) oxide
ii) the iron oxide is, iron (II) oxide, or iron (III) oxide,
iii) the manganese oxide is manganese (II) oxide, manganese (III) oxide or manganese (IV) oxide
iv) the indium oxide, indium (III) oxide
v) the tin oxide is tin (IV) oxide
vi) the vanadium oxide is vanadium (V) oxide.

13. The method according to any of the preceding claims, wherein the electrode is based on manganese dioxide.

14. An electrochemical nucleic acid sequencing apparatus comprising:
a. means for carrying out the steps of annealing and elongating a primer molecule to a template nucleic acid to be sequenced; and
b. an electrochemical cell comprising metal oxides on an electrode being capable of preferentially binding to pyrophosphate.

15. The electrochemical nucleic acid sequencing apparatus according to claim 12, wherein the metal oxides are manganese oxides and the electrode is based on manganese dioxide.

16. Use of an electrode coated with metal oxide preferably manganese oxide, for detecting and or sequencing a nucleic acid or detecting the presence of PPi.
